# EUROPÄISCHE PATENTSCHRIFT

(11) **EP 1 926 407 B1**
(45) Veröffentlichungstag und Bekanntmachung des Hinweises auf die Patenterteilung: **23.11.2016**
(21) Anmeldenummer: 06791630.4
(22) Anmeldetag: 24.08.2006
(51) Int. Cl.: A45D 26/00, A61B 17/54, B26B 19/48, A45D 29/05

(54) **VERFAHREN ZUM ENTFERNEN VON HAAREN, HAARENTFERNUNGSGERÄT UND AUFSATZ HIERFÜR**
SKIN PEELING DEVICE,METHOD FOR MANUFACTURING SUCH A DEVICE AND USE OF SUCH A DEVICE IN A HAND HELD ELECTRICAL APPLIANCE FOR PERSONAL CARE
DISPOSITIF DE DERMABRASION, PROCÉDÉ DE FABRICATION D'UN TEL DISPOSITIF ET UTILISATION D'UN TEL DISPOSITIF DANS UN APPAREIL MANUEL ÉLECTRIQUE DE SOIN PERSONNEL

(30) Priorität: 19.09.2005 DE 102005044737
(43) Veröffentlichungstag der Anmeldung: 04.06.2008
(73) Patentinhaber: Braun GmbH, 61476 Kronberg (DE)
(72) Erfinder: DORBER, Ralf, 61440 Oberursel (DE); SENG, Jürgen, 65779 Kelkheim (DE); GRIESHABER, Frieder, 61267 Neu-Anspach (DE); PEREZ-LOPEZ, Xavier, 65760 Eschborn (DE)
(74) Vertreter: Schneider, Stefan Michael
(86) Internationale Anmeldenummer: PCT/EP2006/008301
(87) Internationale Veröffentlichungsnummer: WO 2007/033746

(56) Entgegenhaltungen:
- EP-A- 0 143 137
- WO-A-2005/058559
- WO-A-2005/108025
- WO-A1-02/056724
- FR-A- 2 641 489
- US-A- 2 936 768
- US-A- 3 081 782

## Beschreibung

Die Erfindung betrifft ein Haarentfernungsverfahren mittels eines in der Hand des Benutzers zu haltenden Haarentfernungsgerätes, welches mindestens zwei Haarentfernungssysteme zum Schneiden und/oder zum Auszupfen von Haaren aufweist. Darüber hinaus betrifft sie ein Haarentfernungsgerät und ein Aufsatzteil für ein Haarentfernungsgerät.

Aus der EP 1 225 818 B1 ist ein Epilationsgerät bekannt, welches mit einem Aufsatzteil verbunden werden kann, das ein Schleifelement aufweist. Dieses Schleifelement lässt sich durch den Antrieb des Enthaarungsgerätes in Schwingung versetzen. Dieses Enthaarungsgerät stellt somit eine Zusatzfunktion zur Verfügung, so dass dieses Gerät entweder als Epilationsgerät oder als Schleifgerät für die Haut verwendbar ist.

Aus der US 2 936 768 ist eine Manikürvorrichtung bekannt, welche einen Zweifacharbeitskopf aufweist, bei dem die beiden Arbeitseinheiten jeweils oszillierend angetrieben werden. Dabei ist eine Einheit als Rasierkopf und die andere parallel dazu angeordnete Einheit als Maniküreinheit ausgebildet. Dadurch soll ein besonders vielseitig einsetzbares Gerät für den täglichen Bedarf geschaffen werden. Diese beiden bekannten Dokumente sehen jeweils nur den Einsatz des einen oder des anderen Arbeitselementes vor, also entweder den Einsatz der Haarentfernungseinheit oder den Einsatz der Schleif-/Maniküreinheit.

Dies bedeutet beispielsweise für den Benutzer eines Enthaarungsgerätes gemäß der EP 1 225 818 B1, dass nach dem Epilationsvorgang die sogenannte Abreibungsvorrichtung auf das Enthaarungsgerät montiert werden muß, um anschließend in einem separaten Arbeitsgang die Hautbehandlung mit der Schleifoberfläche vorzunehmen. In Bezug auf Komfort und Zeitaufwand stellt dies einen beträchtlichen Nachteil dar.

Aus der WO-A1-2002-056724 ist ein Epilationsgerät bekannt, dass parallel zur Epilierwalze mehrere angetriebene Vorsprünge aufweist. Diese Vorsprünge können mit einer abnehmbaren Kappe aus einem Schleifmittel bedeckt sein, um einen Haut-Peeling Effekt zu erreichen.

Der Erfindung liegt daher die Aufgabe zugrunde, ein Verfahren zur Haarentfernung, ein Haarentfernungsgerät bzw. ein Aufsatzteil hierzu zu schaffen, mit welchem auf besonders einfache und für den Anwender bequemer Weise eine besonders gründliche Haarentfernung und Hautbehandlung ermöglicht ist. Dabei wird durch das Abschleifen der Haut mittels der Schleifoberfläche nicht nur die Haut selbst geglättet, es werden auch eventuell nach der Haarentfernung noch vorhandene, wenn auch nur sehr kurze Haare mechanisch in dem Sinne bearbeitet, dass sie an- bzw. abgeschliffen werden. Beispielsweise nach einer Rasur noch verbleibende sehr kurze Haare können an deren Schnittebene angeschliffen und somit verrundet werden, was die Gesamtglattheit der Hautoberfläche deutlich erhöht.

Diese Aufgabe wird erfindungsgemäß durch ein Haarentfernungsgerät mit der Merkmalskombination gemäß Patentanspruch 1, durch ein Aufsatzteil für ein Haarentfernungsgerät gemäß der Merkmalskombination des Patentanspruches 11 bzw. durch ein Verfahren zum Entfernen von Haaren gemäß der Merkmalskombination des Patentanspruches 17 gelöst. Durch die erfindungsgemäße Lösung wird es ermöglicht, in einem einzigen Arbeitsgang sowohl die Haarentfernung als auch die Hautglättung durchzuführen. Ein aufwendiger und zeitraubender Umbau des elektrischen Gerätes entfällt genauso wie ein erneuter Bearbeitungsdurchgang nach Montage der Schleifeinrichtung.

Dabei ist es grundsätzlich möglich, dass dem Schleifkörper ein eigener zusätzlicher Antrieb zugeordnet ist. Gemäß einer bevorzugten Ausführungsform der Erfindung ist jedoch vorgesehen, dass der Schleifkörper Kopplungsmittel aufweist, die mit korrespondierenden, am Haarentfernungsgerät vorgesehenen und mit der Antriebseinrichtung verbundenen weiteren Kopplungsmitteln in Eingriff bringbar sind. Dadurch wird mit einfachen Mitteln der ohnehin vorhandene Antrieb im Haarentfernungsgerät auch für den Betrieb des Schleifkörpers verwendet.

Vorzugsweise ist das erste Haarentfernungssystem ein Scherkopf zum Schneiden von Haaren. Allerdings bietet das in einen Arbeitsgang integrierte Schleifen der Hautoberfläche auch einen Vorteil im Zusammenhang mit der Epilation, so dass eine weitere Ausführungsform der Erfindung vorsieht, dass das erste Haarentfernungsystem ein Epilationskopf zum Auszupfen von Haaren ist.

Um pro Zug des Haarentfernungsgerätes über die Haut sowohl in Bezug auf die Haarentfernung als auch im Bezug auf die Hautglättung besonders effektiv wirken zu können, ist bei der Erfindung vorgesehen, dass das Haarentfernungsgerät mindestens zwei Haarentfernungssysteme aufweist. Dabei ist es besonders vorteilhaft, wenn das erste Haarentfernungssystem als ein Kurzhaarschneidsystem und das zweite Haarentfernungssystem als ein Langhaarschneidsystem ausgebildet ist, wobei das Kurzhaarschneidsystem zwischen dem Schleifkörper und dem Langhaarschneidsystem angeordnet ist. Dadurch werden, wenn das Haarentfernungsgerät mit dem Langhaarschneidsystem voraus über die Haut bewegt wird, zunächst eine Vorkürzung längerer Haare vorgenommen, bevor die verbleibenden Haare mit dem Kurzhaarschneidsystem ausrasiert werden. Anschließend wird die rasierte Fläche durch den sich bewegenden Schleifkörper beaufschlagt und es wird die Haut endgültig geglättet. Für den entsprechenden Anwendungsfall ist es auch sinnvoll, wenn das erste Haarentfernungssystem als Epilationssystem und das zweite Haarentfernungssystem als ein Langhaarschneidsystem ausgebildet ist, wobei dann das Epilationssystem zwischen dem Schleifkörper und dem Langhaarschneidsystem angeordnet ist. Hierbei werden dann bei entsprechender Bewegungsrichtung des Haarentfernungssystemes relativ zur Hautoberfläche zunächst die Haare auf ein bestimmtes Maß vorgekürzt, so dass sie vom Epilationssystem leicht und nahe der Haarwurzel erfasst werden können, wodurch eine Reduktion des während des Auszupfens möglicherweise auftretenden Schmerzes erzielt wird. Dem Auszupfvorgang folgt dann während eines einzigen Bearbeitungszuges dann auch noch das Glätten der Haut mittels des Schleifkörpers.

Der Schleifkörper ist vorteilhafterweise oszillierend angetrieben. Dies begünstigt ein leichtes Entlangführen des Haarentfernungsgerätes auf der Haut. Unerwünschte, störende Einflüsse auf das Handling des Haarentfernungsgerätes bedingt durch Drehmomente und durch die Interaktion einer rotierenden Schleifscheibe mit der Haut können dabei von vorneherein ausgeschlossen werden.

Sind am Aufsatzteil Führungs- bzw. Anlageflächen zum Anlegen an die Haut ausgebildet, so lässt sich das exakte Steuern des Haarentfernungsgerätes entlang einer gewünschten Bahn auf der Haut noch weiter erleichtern.

Im Rahmen der Erfindung ist es auch möglich, dass sowohl der Schleifkörper als auch das zweite Haarentfernungssystem im Aufsatzteil angeordnet ist. Somit ist es auch möglich, einen sogenannten Einkopfrasierer oder Epilierer, also ein Haarentfernungssystem mit lediglich einem aktiven Haarentfernungssystem, durch das Anlegen des Aufsatzteiles derart aufzurüsten, dass es anschließend über insgesamt drei aktive Systeme verfügt. Insbesondere ist dann das zweite Haarentfernungssystem als Langhaarschneidsystem ausgebildet. Idealerweise sind dann die aktiven Elemente im Aufsatzteil derart angeordnet, dass nach dem Aufsetzen des Aufsatzteiles dass dem Haarentfernungsgerät zugeordnete erste Haarentfernungssystem zwischen den beiden im Aufsatzteil angeordneten Systemen, nämlich dem Schleifkörper und dem zweiten Haarentfernungssystem angeordnet ist.

Die obenstehend am Beispiel des Haarentfernungsgerätes ausgeführten Weiterbildungen der Erfindung und die damit verbundenen Vorteile gelten entsprechend auch für diejenigen Unteransprüche, die auf das Aufsatzteil als solches rückbezogen sind.

Das erfindungsgemäße Verfahren zum Entfernen von Haaren der menschlichen Haut mittels eines in der Hand des Benutzers zu haltenden Haarentfernungsgerätes zeichnet sich dadurch aus, dass das Haarentfernungsgerät ein Gehäuse und mindestens ein erstes Haarentfernungssystem zum Schneiden und/oder zum Auszupfen von Haaren aufweist und dass das Haarentfernungssystem in Kontakt mit der Haut gebracht und entlang dieser in einer Vorschubrichtung bewegt wird, wobei dem Haarentfernungssystem in Bezug auf die Vorschubrichtung ein Schleifkörper zum Abschleifen der Haut nachgeschaltet ist, der ebenfalls in Kontakt mit der Haut gebracht ist. Dabei ist es besonders vorteilhaft, wenn der Schleifkörper eine Relativbewegung zum Gehäuse ausführt, um durch diese der Vorschubbewegung überlagerte Schleifbewegung den Wirkungsgrad der Schleifeinrichtung zu verbessern.

Eine bevorzugte Ausführungsform dieses Verfahrens sieht vor, dass als erstes Haarentfernungssystem ein Schersystem verwendet wird. Eine andere Ausführungsform der Erfindung sieht die Verwendung eines Epilationssystems als erstes Haarentfernungssystem vor. Zur Effizienzsteigerung sieht das erfindungsgemäße Verfahren vor, dass dem ersten Haarentfernungssystem in Vorschubrichtung des Haarentfernungsgerätes gesehen ein zweites Haarentfernungssystem vorgeschaltet ist, welches bevorzugt als Langhaarschneidsystem ausgebildet ist.

Weitere Ziele, Merkmale, Vorteile und Anwendungsmöglichkeiten der vorliegenden Erfindung ergeben sich aus der nachfolgenden Beschreibung der Ausführungsbeispiele. Dabei bilden alle beschriebenen oder bildlich dargestellten Merkmale für sich oder in beliebiger Kombination den Gegenstand vorliegender Erfindung, auch unabhängig von ihrer Zusammenfassung in den Ansprüchen oder deren Rückbeziehung.

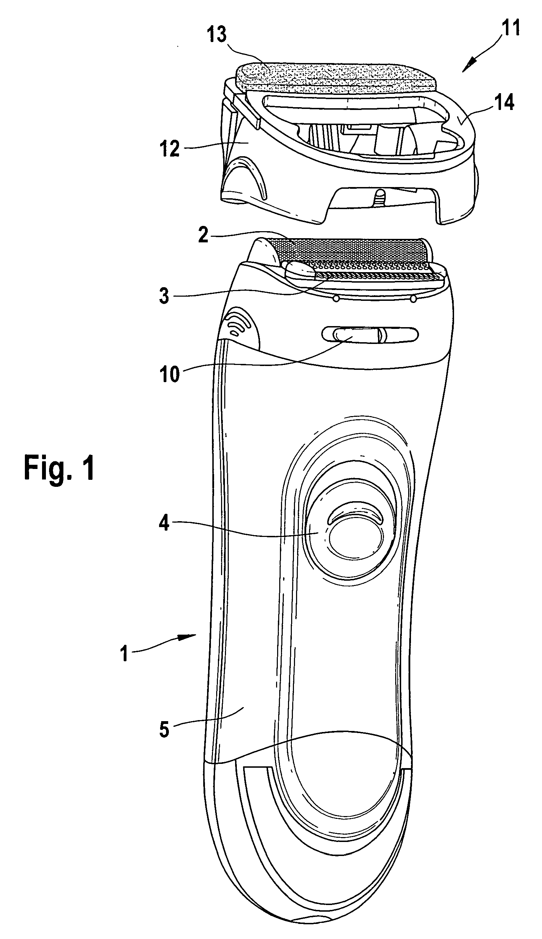

Hierzu zeigt
- Fig. 1: das Haarentfernungsgerät mit abhobenem Aufsatzteil,
- Fig. 2: das Haarentfernungsgerät mit angebrachtem und einsatzbereitem Aufsatzteil,
- Fig. 3: zeigt eine Seitenansicht des Haarentfernungsgerätes,
- Fig. 4: stellt in vergrößerter Ansicht eine Draufsicht auf das Aufsatzteil dar und
- Fig. 5: zeigt einen Schnitt durch das Aufsatzteil und den oberen Bereich des Haarentfernungsgerätes bei aufgesetztem Aufsatzteil.

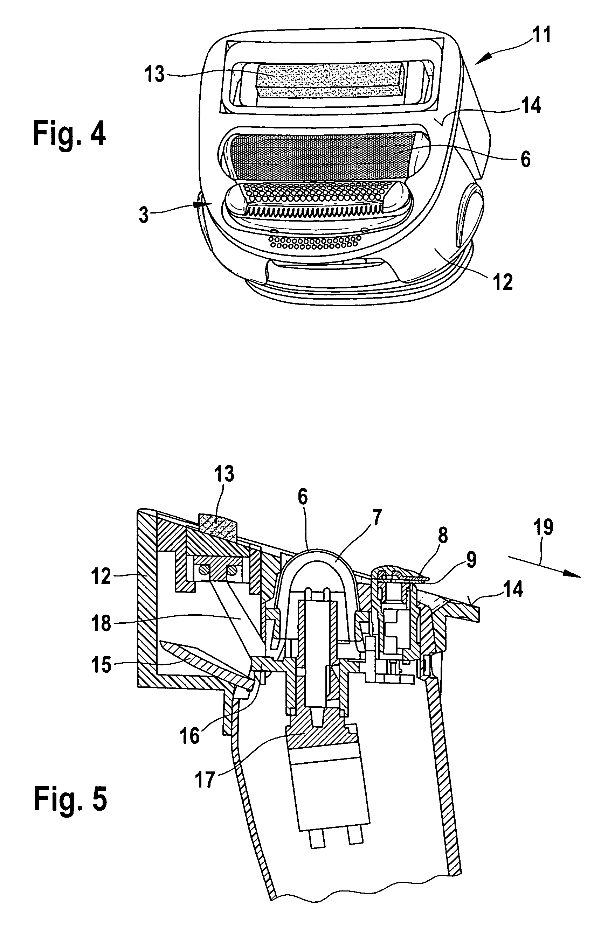

Das Haarentfernungsgerät 1 ist als solches bereits bekannt und wird als Braun LS 5500, Type 5328 vertrieben. Es weist ein Kurzhaarschneidsystem 2 und ein Langhaarschneidsystem 3 auf, die ebenso wie der Ein-/Aus-Schalter 4 auf einem Gehäuse 5 angeordnet sind. In dem Gehäuse 5 ist auch die Stromversorgung und ein Elektromotor vorgesehen, die dem oszillierenden Antrieb der Scherteile dienen. Dies ist der Übersichtlichkeit wegen zeichnerisch nicht dargestellt. Das Kurzhaarschneidsystem besteht in an sich bekannter Weise aus einer das Obermesser bildenden Scherfolie 6, unter welcher ein einer Vielzahl von Klingen aufweisendes Untermesser 7 oszillierend bewegt wird. Das Langhaarschneidsystem 3 wiederum besitzt einen stationären Scherkamm 8, der das Obermesser bildet, und ein Untermesser, welches durch eine oszillierend angetriebene Scherklinge 9 gebildet ist. Diese Teile der Schersysteme sind in Fig. 5 näher dargestellt.

Das Langhaarschneidsystem 3 ist insgesamt elastisch nach oben vorgespannt, und schwimmend gelagert, so dass es leicht der Hautkontur folgen kann. Es kann jedoch durch den Verriegelungsschalter 10 festgesetzt werden, so dass es für die Trimmfunktion besser eingesetzt werden kann.

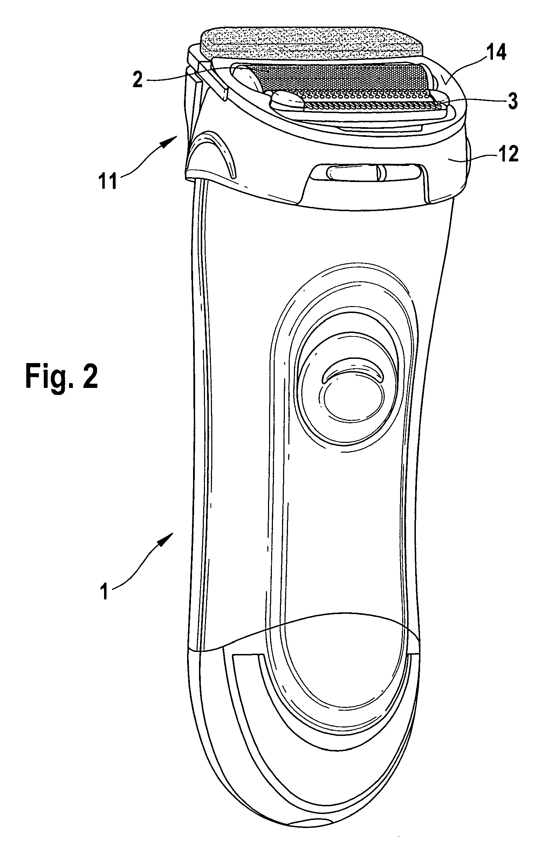

Das Aufsatzteil 11 besitzt einen ringförmigen Tragkörper 12, in welchem der Schleifkörper 13 derart gelagert ist, dass er entlang seiner Längsachse oszillierend angetrieben werden kann. Der Schleifkörper 13 kann als Bimsstein, als Schleifpapier, als offenporiger Sinterblock oder dergleichen ausgeführt sein. Zur Montage des Aufsatzteiles 11 wird dasselbe über den Scherkopf des Haarentfernungsgerätes 1 gestülpt, so dass es mit dem Gehäuse 5 des Haarentfernungsgerätes 1 fest verbunden ist, die Scherelemente, d.h. das Kurzhaarschneidsystem 2 und das Langhaarschneidsystem 3 liegen dabei weiterhin frei zugänglich, so dass sie entsprechend eingesetzt werden können. Sowohl das Kurzhaarschneidsystem 2 als auch das Langhaarschneidsystem 3 überragen bei montiertem Aufsatzteil 11 die am Tragkörper 12 ausgebildete im wesentlichen ringförmig umlaufende Anlagefläche 14 nach oben. Dies kann den Fig. 2 und 5 deutlich entnommen werden. Der Tragkörper 12 besitzt an seiner Vorderseite eine Ausnehmung für den Verriegelungsschalter 10, so dass dieser auch nach Aufsatz des Aufsetzteils 11 zugänglich und bedienbar bleibt.

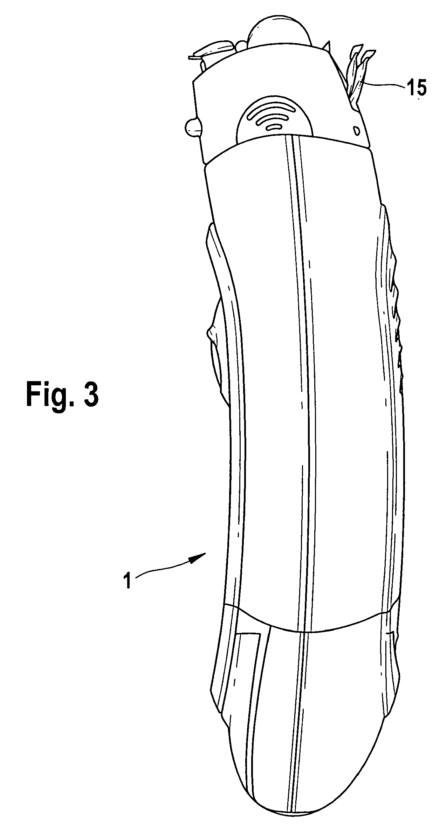

In Fig. 3 ist eine Seitenansicht des Haarentfernungsgerätes 1 dargestellt, in welcher die Klappe 15, welche im Bereich des Scherkopfes an der Rückseite des Haarentfernungsgerätes angeordnet ist, in geöffneter Position gezeigt ist. Diese Klappe 15, welche um eine Achse, die parallel zur Längserstreckung der Scherelemente bzw. des Schleifkörpers 13 verläuft, verschwenkt werden kann, ermöglicht ein Abdecken oder Freilegen der gabelförmigen Kupplung 16, die innerhalb des Gehäuses 5 vorgesehen und mit der Schwingbrücke 17 des Antriebs der Scherelemente 7 bzw. 9 verbunden ist. Die Klappe 15 muß vor den Aufsätzen des Aufsatzteiles 11 geöffnet werden, damit während des Aufsetzvorgangs der mit dem Schleifkörper 13 verbundene Kupplungsfinger 18 in Eingriff mit der Kupplung 16 gebracht werden kann. Sobald dieser Eingriff hergestellt ist, kann die oszillierende Bewegung der Schwingbrücke 17 über die Kupplung 16 und den Kupplungsfinger 18 auf den Schleifkörper 13 übertragen werden, der entlang seiner Längsachse verschiebbar im Tragkörper 12 gelagert ist. Durch entsprechende Ausgestaltung der Klappe und Abstimmung der geometrischen Verhältnisse mit dem Kupplungsfinger 18 bzw. dem Tragkörper 12 kann auch dafür gesorgt werden, dass sich die Klappe 15 beim Aufsetzen des Aufsatzteiles 11 auf das Haarentfernungsgerät 1 automatisch öffnet.

Wird nun das Haarentfernungsgerät 1 mit aufgesetztem Aufsatzteil 11 mit der zu bearbeitenden Hautfläche in Kontakt gebracht und in Vorschubrichtung 19, d.h. in Richtung des Langhaarschneidsystems 3 über dieselbe geführt, so werden durch das Langhaarschneidsystem 3 zunächst auf der Haut befindliche längere Haare gekürzt, die dann vom nachfolgenden Kurzhaarschneidsystem 2 ausrasiert werden. Anschließend wird die rasierte Haut durch den sich oszillierend hin und herbewegenden Schleifkörper 13 endgültig geglättet, wobei auch beispielsweise ausgetrocknete oder abgestorbene Hautpartikel oder Verhornungen entfernt werden. Sämtliche dieser beschriebenen Vorgänge werden während eines einzigen Bearbeitszuges über die Haut ausgeführt. Soll dagegen lediglich eine Rasur oder ein Trimmen von Haaren vorgenommen werden, so lässt sich das Aufsatzteil 11 leicht vom Haarentfernungsgerät 1 entfernen und es kann in der üblichen Weise angewandt werden.

In einer zeichnerisch nicht dargestellten Ausführungsform der Erfindung ist das Haarentfernungssystem des Haarentfernungsgerätes 1 als Epilationskopf ausgebildet, welches durch entsprechende Klemmelemente auf der Haut befindliche Haare einklemmt und entfernt. Auch bei einem solchen Haarentfernungsgerät ist es wirkungsvoll durch entsprechenden Einsatz eines Aufsatzteils mit darin gelagertem Schleifkörper die Haut nach erfolgter Epilation mittels dieser Schleifvorrichtung zu bearbeiten. Dabei erhöht es den Komfort, wenn zusätzlich zur Epilationsvorrichtung auch noch ein Langhaarschneidsystem, welches dem Epilationssystem vorgeschaltet ist, zum Einsatz gebracht wird, wobei der Schleifkörper 13 dem Epilationssystem folgt. In besonders vorteilhafter Weise können dann mit einem einzigen Arbeitsgang bzw. -zug auf der Haut befindliche Haare zunächst durch ein Langhaarschneidsystem auf eine bestimmte Länge vorgekürzt werden, welche es dem nachfolgenden Epilationsgerät ermöglicht, diese dann effektiv und möglichst schmerzfrei zu ergreifen und auszuzupfen, während im letzten integrierten Arbeitsschritt dann die enthaarte Haut mit der Schleifvorrichtung bearbeitet wird.

## Patentansprüche

1. Ein in der Hand des Benutzers zu haltenden Haarentfernungsgerät (1) mit einem Gehäuse (5), einer Antriebseinrichtung zum Antrieb mindestens eines ersten Haarentfernungssystems (2) zum Schneiden und/oder zum Auszupfen von Haaren und einem nahe diesem angeordneten ebenfalls angetriebenen, in Kontakt mit der Haut zu bringenden Schleifkörper (13), wobei der Schleifkörper (13) in einem als zusätzlich zum ersten Haarentfernungssystem (2) an das Gehäuse (5) anbringbaren bzw. entfernbaren Aufsatzteil (11) angeordnet und gelagert ist und wobei dem ersten Haarentfernungssystem (2) in Vorschubrichtung (19) gesehen ein zweites Haarentfernungssystem (3) vorgeschaltet und der Schleifkörper nachgeschaltet ist.

2. Haarentfernungsgerät nach Anspruch 1, **dadurch gekennzeichnet, dass** der Schleifkörper (13) Kopplungsmittel (18) aufweist, die mit korrespondierenden am Haarentfernungsgerät (1) vorgesehenen und mit der Antriebseinrichtung (17) verbundenen weiteren Kopplungsmittels (16) in Eingriff bringbar sind.

3. Haarentfernungsgerät nach einem der vorhergehenden Ansprüche, **dadurch gekennzeichnet, dass** das erste Haarentfernungssystem (1) ein Scherkopf zum Schneiden von Haaren ist.

4. Haarentfernungsgerät nach Anspruch 1 oder 2, **dadurch gekennzeichnet, dass** das erste Haarentfernungssystem ein Epilationskopf zum Auszupfen von Haaren ist.

5. Haarentfernungsgerät nach einem der vorhergehenden Ansprüche, **dadurch gekennzeichnet, dass** mindestens zwei Haarentfernungssysteme (2, 3) vorgesehen sind.

6. Haarentfernungsgerät nach Anspruch 5, **dadurch gekennzeichnet, dass** das erste Haarentfernungssystem (2) als ein Kurzhaarschneidsystem und das zweite Haarentfernungssystem (3) als ein Langhaarschneidsystem ausgebildet ist, wobei das Kurzhaarschneidsystem (2) zwischen dem Schleifkörper (13) und dem Langhaarschneidsystem (3) angeordnet ist.

7. Haarentfernungsgerät nach einem der vorhergehenden Ansprüche, **dadurch gekennzeichnet, dass** der Schleifkörper (13) oszillierend angetrieben ist.

8. Haarentfernungsgerät nach einem der vorhergehenden Ansprüche, **dadurch gekennzeichnet, dass** am Aufsatzteil Führungs- bzw. Anlageflächen (14) zum Anlegen an die Haut ausgebildet sind.

9. Haarentfernungsgerät nach einem der vorhergehenden Ansprüche, **dadurch gekennzeichnet, dass** sowohl der Schleifkörper (13) als auch das zweite Haarentfernungssystem (3) im Aufsatzteil angeordnet ist.

10. Haarentfernungsgerät nach einem der vorhergehenden Ansprüche, **dadurch gekennzeichnet, dass** Haarentfernungssystem (2) bzw. die Haarentfernungssysteme (2,3) und/oder der Schleifkörper (13) abtauchbar gelagert und elastisch in Richtung auf die Haut vorgespannt sind.

11. Aufsatzteil für ein in der Hand des Benutzers zu haltenden Haarentfernungsgerät mit einem ersten und einem zweiten Haarentfernungssystem zum Schneiden und/oder zum Auszupfen von Haaren wobei im Aufsatzteil (11) ein antreibbarer, in Kontakt mit der Haut zu bringenden Schleifkörper (13) zum Abschleifen der Haut angeordnet ist und das Aufsatzteil am Gehäuse (5) des Haarentfernungsgerätes (1) derart anbringbar ist, dass dem ersten Haarentfernungssystem (2) in Vorschubrichtung (19) gesehen das zweite Haarentfernungssystem (3) vorgeschaltet und der Schleifkörper (13) nachgeschaltet ist.

12. Aufsatzteil nach Anspruch 11, **dadurch gekennzeichnet, dass** der Schleifkörper (13) mit Ankoppelmitteln (18) versehen ist, der mit korrespondierenden weiteren Kopplungsmitteln (16) des Haarentfernungsgerätes (1) in Eingriff bringbar sind.

13. Aufsatzteil nach einem der Ansprüche 11 oder 12, **dadurch gekennzeichnet, dass** der Schleifkörper (13) oszillierend angetrieben ist.

14. Aufsatzteil nach einem der Ansprüche 11 bis 13, **dadurch gekennzeichnet, dass** am Aufsatzteil (11) Führungs- bzw. Anlageflächen (14) zum Anlegen an die Haut ausgebildet sind.

15. Aufsatzteil nach einem der Ansprüche 11 bis 14, **dadurch gekennzeichnet, dass** sowohl der Schleifkörper (13) als auch ein zweites Haarentfernungssystem (3) im Aufsatzteil (11) angeordnet ist.

16. Aufsatzteil nach einem der Ansprüche 11 bis 15, **dadurch gekennzeichnet, dass** der Schleifkörper (23) und/oder das zweite Haarentfernungssystem (3) abtauchbar im Aufsatzteil (11) gelagert und elastisch in Richtung auf die Haut vorgespannt ist.

17. Verfahren zum Entfernen von Haaren aus der menschlichen Haut mittels eines in der Hand des Benutzers zu haltenden Haarentfernungsgerätes (1), wobei das Haarentfernungsgerät (1) ein Gehäuse (5) und mindestens ein erstes Haarentfernungssystem (2) zum Schneiden und/oder zum Auszupfen von Haaren aufweist und wobei das Haarentfernungssystem in Kontakt mit der Haut gebracht und entlang dieser in eine Vorschubrichtung (19) bewegt wird, wobei dem Haarentfernungssystem (2) in Bezug auf die Vorschubrichtung ein Schleifkörper (13) zum Abschleifen der Haut nachgeschaltet ist, der ebenfalls in Kontakt mit der Haut gebracht ist und wobei dem ersten Haarentfernungssystem (2) in Vorschubrichtung (19) gesehen ein zweites Haarentfernungssystem (3) vorgeschaltet ist.

18. Verfahren nach Anspruch 17, **dadurch gekennzeichnet, dass** der Schleifkörper (13) eine Relativbewegung zum Gehäuse (5) ausführt.

19. Verfahren nach einem der Ansprüche 17 und 18, **dadurch gekennzeichnet, dass** das erste Haarentfernungssystem (2) ein Scherkopf zum Schneiden von Haaren ist.

20. Verfahren nach einem der Ansprüche 17 und 18, **dadurch gekennzeichnet, dass** das erste Haarentfernungssystem (2) ein Epilationskopf zum Auszupfen von Haaren ist.

21. Verfahren nach einem der Ansprüche 17 bis 20, **dadurch gekennzeichnet, dass** das zweite Haarentfernungssystem (3) ein Langhaarschneidsystem ist.

## Claims

1. A hand-held hair removal apparatus (1) having a housing (5), a drive mechanism for driving at least one first hair removal system (2) for cutting and/or for plucking out hairs and an abrasive body (13) arranged close to same that is also driven and to be brought into contact with the skin, the abrasive body (13) being arranged and mounted in an attachment part (11) that can be attached to or removed from the housing (5) in addition to the first hair removal system (2) and a second hair removal system (3) being arranged upstream from the first hair removal system (2) as seen in the feed direction (19) and the abrasive body being arranged downstream.

2. The hair removal device according to claim 1, **characterized in that** the abrasive body (13) has coupling means (18) that can be engaged with corresponding further coupling means (16) that are provided on the hair removal apparatus (1) and connected with the drive mechanism (17).

3. The hair removal apparatus according to any of the preceding claims, **characterized in that** the first hair removal system (1) is a shaving head for cutting hairs.

4. The hair removal apparatus according to claim 1 or 2, **characterized in that** the first hair removal system is an epilation head for plucking out hairs.

5. The hair removal apparatus according to any of the preceding claims, **characterized in that** at least two hair removal systems (2, 3) are provided.

6. The hair removal apparatus according to claim 5, **characterized in that** the first hair removal system (2) is designed as a short hair cutting system and the second hair removal system (3) is designed as a long hair cutting system, the short hair cutting system (2) being arranged between the abrasive body (13) and the long hair cutting system (3).

7. The hair removal apparatus according to any of the preceding claims, **characterized in that** the abrasive body (13) is driven in an oscillating manner.

8. The hair removal device according to any of the preceding claims, **characterized in that** guide or contact surfaces (14) for application to the skin are formed on the attachment part.

9. The hair removal apparatus according to any of the preceding claims, **characterized in that** both the abrasive body (13) and the second hair removal system (3) are arranged in the attachment part.

10. The hair removal apparatus according to any of the preceding claims, **characterized in that** the hair removal system (2) or the hair removal systems (2, 3) and/or the abrasive body (13) are mounted in a submersible manner and elastically biased towards the skin.

11. An attachment for a hand-held hair removal apparatus having a first and a second hair removal device for cutting and/or for plucking out hairs, an abrasive body (13) for abrading the skin that is capable of being driven and to be brought into contact with the skin being arranged in the attachment part (11) and the attachment part being attachable to the housing (5) of the hair removal apparatus (1) to such an extent that the second hair removal system (3) is arranged upstream from the first hair removal system (2) as seen in the feed direction (19) and the abrasive body (13) is arranged downstream.

12. The attachment part according to claim 11, **characterized in that** the abrasive body (13) is provided with coupling means (18) that can be engaged with corresponding further coupling means (16) of the hair removal apparatus (1).

13. The attachment part according to either of the claims 11 or 12, **characterized in that** the abrasive body (13) is driven in an oscillating manner.

14. The hair removal device according to any of the claims 11 to 13, **characterized in that** guide or contact surfaces (14) for application to the skin are formed on the attachment part (11).

15. The attachment part according to any of the claims 11 to 14, **characterized in that** both the abrasive body (13) and a second hair removal system (3) are arranged in the attachment part (11).

16. The attachment part according to any of the claims 11 to 15, **characterized in that** the abrasive body (23) and/or the second hair removal system (3) is mounted in a submersible manner in the attachment part (11) and elastically biased towards the skin.

17. A method for removing hairs from human skin using a hand-held hair removal apparatus (1), the hair removal apparatus (1) having a housing (5) and at least a first hair removal system (2) for cutting and/or plucking out hairs and the hair removal system being brought into contact with the skin and moved along same in a feed direction (19), an abrasive body (13) for abrading the skin being arranged downstream from the hair removal system (2) with respect to the feed direction, said abrasive body also being brought into contact with the skin and a second hair removal system (3) being arranged upstream from the first hair removal system (2) as seen in the feed direction (19).

18. The method according to claim 17, **characterized in that** the abrasive body (13) performs a relative movement with respect to the housing (5).

19. The method according to any of the claims 17 and 18, **characterized in that** the first hair removal system (2) is a shaving head for cutting hairs.

20. The method according to any of the claims 17 and 18, **characterized in that** the first hair removal system (2) is an epilation head for plucking out hairs.

21. The method according to any of the claims 17 to 20, **characterized in that** the second hair removal system (3) is a long hair cutting system.

## Revendications

1. Épilateur (1) devant être tenu dans la main d'un utilisateur, doté d'un boîtier (5), d'un dispositif d'entraînement servant à entraîner au moins un premier système d'épilation (2) pour la coupe et/ou l'extirpation des poils et d'un corps abrasif (13) disposé à côté du système et également entraîné, destiné à être placé en contact avec la peau, le corps abrasif (13) étant disposé et monté dans un accessoire (11) pouvant être placé de façon montable et démontable sur le boîtier (5) en plus du premier système d'épilation (2) et un deuxième système d'épilation (3) étant prévu en amont du premier système d'épilation (2) de la manière observée dans la direction d'avancée (19) et en aval du corps abrasif.

2. Épilateur selon la revendication 1, **caractérisé en ce que** le corps abrasif (13) présente des moyens d'accouplement (18), qui peuvent être mis en prise avec d'autres moyens d'accouplement (16) correspondants prévus sur l'épilateur (1) et reliés avec le dispositif d'entraînement (17).

3. Épilateur selon une des revendications précédentes, **caractérisé en ce que** le premier système d'épilation (1) est une tête de rasage pour la coupe des poils.

4. Épilateur selon la revendication 1 ou 2, **caractérisé en ce que** le premier système d'épilation est une tête d'épilation pour l'extirpation des poils.

5. Épilateur selon une des revendications précédentes, **caractérisé en ce qu'**au moins deux systèmes d'épilation (2, 3) sont prévus.

6. Épilateur selon la revendication 5, **caractérisé en ce que** le premier système d'épilation (2) est formé comme un système de coupe des poils courts et **en ce que** le deuxième système d'épilation (3) est formé comme un système de coupe des poils longs, le système de coupe des poils courts (2) étant disposé entre le corps abrasif (13) et le système de coupe des poils longs (3).

7. Épilateur selon une des revendications précédentes, **caractérisé en ce que** le corps abrasif (13) est entraîné en oscillation.

8. Épilateur selon une des revendications précédentes, **caractérisé en ce que** des surfaces d'appui ou de guidage (14) pour s'appuyer sur la peau sont formés sur l'accessoire.

9. Épilateur selon une des revendications précédentes, **caractérisé en ce que** le corps abrasif (13) mais également le deuxième système d'épilation (3) sont disposés dans l'accessoire.

10. Épilateur selon une des revendications précédentes, **caractérisé en ce que** le système d'épilation (2) ou les systèmes d'épilation (2, 3) et/ou le corps abrasif (13) sont montés de façon rétractable et sont pré-contraints de façon élastique en direction de la peau.

11. Accessoire pour un épilateur devant être tenu dans la main d'un utilisateur, avec un premier et un deuxième système d'épilation pour la coupe et/ou l'extirpation de poils, un corps abrasif (13) pouvant être entraîné et devant être mis en contact avec la peau pour abraser la peau étant agencé dans l'accessoire (11) et l'accessoire pouvant être monté sur le boîtier (5) de l'épilateur (1) de telle sorte que le premier système d'épilation (2) dans la direction d'avancée (19) soit placé en amont du deuxième système d'épilation (3), de la manière observée dans la direction d'avancée et en aval du corps abrasif (13).

12. Accessoire selon la revendication 11, **caractérisé en ce que** le corps abrasif (13) est doté de moyens d'accouplement (18), qui peuvent être mis en prise avec d'autres moyens d'accouplement (16) correspondants de l'épilateur (1).

13. Accessoire selon une des revendications 11 ou 12, **caractérisé en ce que** le corps abrasif (13) est entraîné en oscillation.

14. Accessoire selon une des revendications 11 à 13, **caractérisé en ce que** des surfaces d'appui ou de guidage (14) pour s'appuyer sur la peau sont formés sur l'accessoire (11).

15. Accessoire selon une des revendications 11 à 14, **caractérisé en ce que** le corps abrasif (13) mais également un deuxième système d'épilation (3) sont disposés dans l'accessoire (11).

16. Accessoire selon une des revendications 11 à 15, **caractérisé en ce que** le corps abrasif (23) et/ou le deuxième système d'épilation (3) sont placés de manière rétractable dans l'accessoire (11) et sont pré-contraints de façon élastique en direction de la peau.

17. Procédé d'élimination des poils sur la peau humaine au moyen d'un épilateur (1) devant être tenu dans la main d'un utilisateur, l'épilateur (1) comprenant un boîtier (5) et au moins un premier système d'épilation (2) pour la coupe et/ou l'extirpation des poils et dans lequel le système d'épilation est amené au contact de la peau et déplacé le long de celle-ci dans une direction d'avancée (19), dans lequel le système d'épilation (2) est monté en aval d'un corps abrasif (13) pour abraser la peau, par rapport à la direction d'avancée, qui est également amené en contact avec la peau et dans lequel un deuxième système d'épilation (3) est monté en amont du premier système d'épilation (2) de la manière observée dans la direction d'avancée (19).

18. Procédé selon la revendication 17, **caractérisé en ce que** le corps abrasif (13) réalise un mouvement relatif par rapport au boîtier (5).

19. Procédé selon une des revendications 17 et 18, **caractérisé en ce que** le premier système d'épilation (2) est une tête de rasage pour la coupe des poils.

20. Procédé selon une des revendications 17 et 18, **caractérisé en ce que** le premier système d'épilation (2) est une tête d'épilation pour l'extirpation des poils.

21. Procédé selon une des revendications 17 à 20, **caractérisé en ce que** le deuxième système d'épilation (3) est un système de coupe des poils longs.
